Europäisches Patentamt

European Patent Office

Office européen des brevets

(19)

(11) **EP 0 630 203 B1**

(12) **EUROPEAN PATENT SPECIFICATION**

(45) Date of publication and mention
of the grant of the patent:
**31.07.2002 Bulletin 2002/31**

(21) Application number: **92905626.5**

(22) Date of filing: **28.02.1992**

(51) Int Cl.$^7$: **A61B 5/00**

(86) International application number:
**PCT/CA92/00091**

(87) International publication number:
**WO 93/16629 (02.09.1993 Gazette 1993/21)**

(54) **NON-INVASIVE DEVICE AND METHOD FOR DETERMINING CONCENTRATIONS OF VARIOUS COMPONENTS OF BLOOD OR TISSUE**

VORRICHTUNG UND VERFAHREN ZUR NICHTINVASIVEN MENGENBESTIMMUNG VON IM BLUT ODER GEWEBE VORLIEGENDEN BESTANDTEILEN

DISPOSITIF NON INVASIF ET METHODE POUR DETERMINER LES CONCENTRATIONS DE DIFFERENTS COMPOSANTS DU SANG OU DES TISSUS

(84) Designated Contracting States:
**DE FR GB IT**

(43) Date of publication of application:
**28.12.1994 Bulletin 1994/52**

(73) Proprietor: **CADELL, Theodore E.**
**Waterloo, Ontario N2L 6CI (CA)**

(72) Inventor: **CADELL, Theodore E.**
**Waterloo, Ontario N2L 6CI (CA)**

(74) Representative: **Warren, Anthony Robert et al**
**BARON & WARREN,**
**18 South End,**
**Kensington**
**London W8 5BU (GB)**

(56) References cited:
| | |
|---|---|
| **EP-A- 0 262 779** | **EP-A- 0 282 234** |
| **EP-A- 0 335 357** | **WO-A-89/02718** |
| **WO-A-91/01678** | **US-A- 4 013 832** |
| **US-A- 5 070 874** | |

EP 0 630 203 B1

## Description

This invention relates to a non-invasive device and method for continuously monitoring and measuring concentration levels of blood constituents in humans, using the near infrared portion of the light spectrum and, in particular, relates to a device suitable for continuously, or on demand, monitoring concentration levels of blood contituents within the human body.

Previous devices for non-invasively monitoring concentrations of blood constituents of a patient are known. Usually, a sensor is used to externally measure either the concentration of the constituent in gases emitted by the body; or the concentration contained in perspiration; or the concentration contained in body fluids such as tears, saliva, or urine samples; or, alternatively, the blood constituent is measured using radiation passed through a part of the patient's body such as the earlobe.

However, some of the previous radiation measuring devices, have a radiation source which emits light in one wavelength only and are therefore not accurate or broadly applicable enough for practical use.

Other previous devices (e.g. pulse oxymetry) have more than one light source but have only a limited number (e.g. three) of measuring wavelengths.

In the U.S. Robinson Patent No. 4,975,581, there is described a non-invasive device using a broadband lamp whose light, after passing through the test sample, for example the finger of a patient, is optically coupled through a number of light filters, each with its own designated transmission wavelength, to the collector. While the Robinson patent shows the use of several wavelengths at the source, only a limited number of these wavelengths are collected and only some of those collected actually show differential absorption. With Robinson, fingers of different sizes have a different path length.

Some of these previous devices (e.g. U.S. Rosenthal Patent No. 4,286,327) must measure both the intensity changes in the range of transmission wavelength and the changes in intensity distribution. Further, some previous devices are controlled to take a series of measurements at successively higher or lower wavelengths. This can be extremely time consuming.

Still further, some previous devices do not take into account changes in the thickness of one patient's earlobes (or other body part) compared to that of other patients or the change in size of a patient's earlobes (or other body part) and hence the transmission path length due to the pulsing of blood through the lobe (or other body part).

Some prior devices do not take into account temperature variations in the earlobes (or other body part) from patient to patient, or the results fluctuate with prolonged operation.

Some previous devices do not address the problem of stability of placement of the body part in relation to the optical path of the instrument. It is equally important to control the physical distance between entry and exit points as it is to allow free circulation of blood in the finger during the measurement process (see EP 0 430 340 A2).

EP-A-0 430 340 discloses a non-invasive device for measuring concentration levels of constituents of blood and tissue, which includes a receptor shaped so that the subject i.e. selected body part, to be measured can be placed in contact with the receptor. The receptor also has means for eliminating extraneous light, the receptor having an entrance and an exit for the light from the light source. The entrance and exit are located relative to one another so that the body part covers both entrance and exit.

Light which enters the body part selected is typically scattered, but to varying degrees in different individuals and to varying degrees at different times in the same individual. Allowances must be made for such scattering. Instruments which are based on a single detector placed adjacent to the body part measure only a fraction of the light impinging on the body part. The fraction not measured is partly lost due to absorption and partly lost due to scattering. The part lost due to scattering is not accounted for and this results in errors in estimating the part accounted for by absorption. The farther the detector is located from the body part measured, the greater the error due to scattering unless some independent measurement is made.

The Barnes, et al. U.S. Patent Number 5,070,874 describes a non-invasive method and apparatus for determining the amount of glucose in the blood of a patient. The Byrne, et al. Application WO91/01678 describes an oximeter to measure oxygen non-invasively. The Douglas U.S. Patent Number 4,013,832 describes a solid state image modulator. The Cheung, et al. Application Number EPA-0262779 describes an oximeter using a temperature sensor. The Gowling Application Number EPA-0282234 describes a device and method for detecting and measuring the concentration of a substance in a sample by optoacoustic spectroscopy where readings can be related to the heartbeat to improve the signal-to-noise ratio.

Previous devices are not sufficiently accurate to be used in the actual measurement of blood constituent concentration levels by patients; or, they are designed to measure for one component only and must be physically changed to measure for a different component; or, the device takes an unreasonably long time to produce a result; or, they cannot produce results in an easy-to-use form; or, they cannot measure the results of two or more components simultaneously; or they do not collect measurements at sufficient wavelengths to produce accurate results; or they do not average the results; or the results are not consistent enough when used with patients of widely varying characteristics. Obviously, if the device gives an inaccurate reading, disastrous results could occur for the patient using the device to calculate, for example, dosages for insulin administration. The fundamental requirement of non-invasive devices is that they produce accurate, reliable results with patients of widely varying characteristics so that they can replace invasive techniques. None of the prior art devices has met this requirement.

Invasive techniques of measuring blood constituents are, of course, in common usage. These techniques are painful, potentially dangerous and expensive to operate. The normal procedure is to obtain a blood sample from a vein and this sample is then tested in a medical laboratory, using a number of chemical procedures to measure each constituent separately. Alternatively, home glucose testing uses a finger puncture that is spotted onto an enzyme-based semi-permeable membrane test strip and is allowed to react for a certain length of time with insulin administration, then based upon either a visual colour comparison with a standard colour chart or by means of a more accurate and unambiguous spectroscopic technique (for example reflectance). There is a risk of infection and sometimes a patient can develop a rash when these invasive techniques are used.

It is an object of the present invention to provide a device for monitoring or measuring the concentration levels of one particular constituent or, alternatively, of measuring the concentration level of several different components simultaneously, said device producing results in a short time period that are highly accurate and compare favourably to invasive techniques.

The present invention is directed to a non-invasive device for measuring concentration levels of constituents of blood and tissue in a living human subject, generally of the type disclosed in US-A-4,286,327 and as defined in the precharacterising clause of claim 1.

According to a first aspect of the present invention, there is provided a non-invasive device as defined in the characterising clause of claim 1.

According to a second aspect of the present invention there is provided a non-invasive method for measuring concentration levels of blood and tissue constituents within a living human subject, as defined in claim 29.

Various embodiments of the present invention will now be described, by way of example, with reference to the accompanying drawings, in which:

> Figure 1 is a schematic view showing the basic components of a single beam device for non-invasively monitoring the concentration levels of blood constituents; and
> Figure 2 is a schematic view showing the basic components of a dual beam device for non-invasively continuously monitoring the concentration levels of blood constituents.

The near infrared region of the electromagnetic spectrum is generally considered to be the spectral range extending from 650 nm through to 2700 nm. Absorption bands observed in this range are primarily the combination and overtone bands of the fundamental infrared bands. Although very weak in intensity, being typically less than one-tenth in intensity than those of the fundamental infrared bands, these bands are considered to be quite analytically useful because nearly all chemical species exhibit characteristic absorption bands in this spectral range. The near infrared region is particularly well suited to in vivo diagnostic applications because, in human tissue, there is adequate reduction in absorption of the incident radiation, such that penetration of the radiation through the tissue is sufficient to allow accurate quantitative analysis.

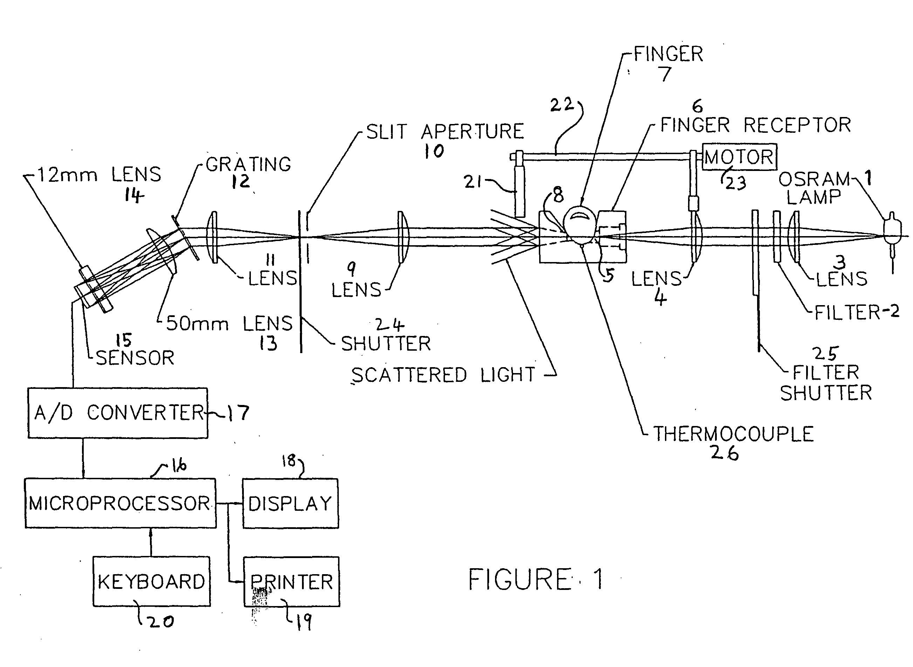

As shown in Figure 1, a non-invasive single beam device for measuring concentration levels of blood constituents has a polychromatic light source 1. The light source can emit light over a very wide bandwidth including light in the near infrared spectrum. The light from said light source 1 passes first through a filter 2 which limits the energy of the light incident on the body part to a range of approximately 650 nm to approximately 1250 nm. Preferably, the filter 2 limits the energy of the light incident on the body part to a range of approximately 700 nm to approximately 1100 nm. The purpose of limiting the light is to eliminate the light rays that heat the body part, thereby allowing a higher intensity bulb to be used as the light source. With a higher intensity bulb, a measurement can be taken more quickly, thereby minimizing the opportunity for movement of the body part. Before passing through said filter 2, the near infrared radiation passes through a collimater 3, which is a lens that concentrates the light into a narrow parallel beam. Said parallel beam is focused by a second lens 4 on the entrance or entry port 5 of a receptor 6. Said receptor is shaped to receive the body part 7, namely a finger. The light passes through the receptor 6 and through the finger and is scattered by said finger. In addition to the entrance 5, the receptor has an exit or exit port 8. The entrance 5 and exit 8 are located relative to one another so that the body part 7 covers both the entrance and the exit. The collimated rays of said scattered light are collected by a third lens 9 and directed through a slit 10 to a fourth lens 11 which collimates said light onto a diffraction or holographic grating 12. The light which passes through the grating is dispersed into its component wavelengths and focused by lenses 13, 14 onto a sensor comprising a linear array detector 15 so that the light in the infrared region falls along the length of said detector. The light could be collected by reflective surfaces rather than lenses. Said linear array detector 15 has a series of diodes (each diode representing one element) and is electronically scanned to measure the charge accumulated on each element. The detector can be a photo diode array. The charge is proportional to the intensity of light falling on each element of said array and hence measures the intensity of light for each wavelength transmitted through the tissue in the receptor 6.

The detector is connected to a microprocessor 16 through an analog to digital converter 17, with the microprocessor analyzing the measurements and ultimately producing a result for each concentration level determined. The result can be shown on a display 18 and/or printed on a printer 19. A keyboard 20 allows a user to control the device, for example, to specify a particular constituent to be measured.

After the measurements are taken for the transmittance, the log of the inverse of these measurements is taken, that is, log (1/T), where T represents the transmittance. For calculation of the absorbence [i.e. log (1/T)], it is necessary to perform a reference measurement of the light incident on the body part. To do this, the finger 7 is removed from the receptor, and the lens 4 which focuses and directs the near infrared light onto the receptor 6 is removed. In the absence of said lens, collimated infrared light is directed into the entrance or entry port 5 of the receptor 6. Since the light passing through the receptor in the absence of a body part is many times more intense than the scattered light collected from said body part, a neutral density filter 21 is placed at the exit or exit port 8 of the receptor so that the intensity of dispersed light incident on the linear array detector does not drive said detector to saturation. The placement and displacement of said lens 4 and neutral density filter 21 are accomplished by attaching them to a common shaft 22 and rotating the shaft with a motor 23.

The linear array detector 15 has several elements, preferably 256, and each element has a slightly different leakage current. To compensate for this leakage current with a predetermined integration time, it is necessary to perform the integration in the absence of light so that the effects of the leakage current can be subtracted. To accomplish this a shutter 24 is placed immediately after the slit 10 to block the light during the dark scans. If different integration times are used for the measurement and reference scans, then dark current measurement must be performed for both integration times.

Once the detector 15 is enabled to integrate the light falling on its various elements, each said element begins to accumulate a charge proportional to the incident light. The rate at which the charge accumulates for each said element is proportional to the intensity of light falling on that element. The intensity of light falling on an element is dependent on the amount of scattering in the finger through which the light passes. The greater the degree of scattering, the less light is collected for the collimator lens 11 which passes light onto the diffraction grating 12 and thence onto the detector 15. To measure the degree of scattering it is only necessary to integrate for a long enough period of time for the analog to digital conversion (ADC) value for the wavelength at which the peak light throughput occurs to fall within a specified range of analog to digital conversion (ADC) values. This may be accomplished by starting with an arbitrary or average integration time. If the light throughput is so great as to saturate the detector at the chosen wavelength, the ADC value will be at a maximum, in which case the integration time is halved and another measurement is made. When the ADC is less than the specified range (e.g. 12000 to 14000 for a maximum value for the converter 17 of 16384 or approximately 73% to approximately 85% of the maximum value) a calculation is made to determine the integration time necessary to place the ADC value within the specified range. A measurement is then made and the integration time and ADC value are saved to memory for that measurement. By dividing the obtained ADC value by the integration time (IT) and taking the log of the quotient, a value is obtained which has a strong relationship to the amount of scattering. This value, log (ADC/IT), can then be used for multiplicative scatter correction or can be introduced as a variable in calculating the concentrations of analytes.

For wavelength and absorbence calibration of the instrument, a shutter 25 is inserted in the collimated beam preceding the finger receptor 6. This shutter positions a calibration filter (e.g. dydimium) so that well known absorbence peaks present in the filter can be routinely used to calibrate for wavelength and absorbence accuracy.

The polychromatic light source 1 can be a quartz-halogen or a tungsten halogen bulb and may be powered by a stabilized power source, for example, a DC power supply, or by a battery. It should be noted that, after activation of the light source 1, the scanning detector 15 is read so that light is passed through the receptor 6 and measured by the detector 15 by taking a series of measurements on a selected wavelength. Measurements on this wavelength are sensitive to increased plethysmographic volume, or in other words, path length, and so measure the heart pressure pulse. The microprocessor control activates the scanning of the linear array detector 15 only after a detected pulse has occurred and the full spectrum measurements are then taken for the light after it passes through the receptor 6. Scanning is stopped when another pulse is detected on the selected wavelength. In other words, measurements are taken only when the blood pressure in the finger 7 is at a constant level. This ensures that the path length of the tissue through which the light passes will be uniform.

In a further variation, the device can take measurements regardless of the pulse of the subject. The microprocessor 16 can then be controlled by computer software to select those measurements that are taken between pulses and to base the calculation of the concentration levels on the selected measurements. In a still further variation, the measurement upon which the results are based could be taken during pulses.

In relation to said pulse, it is possible to collect data at the peak of said pulse as well as between said pulses. In this way the difference between the two measurements would be absorption attributable to blood constituents only. There are various means for detecting a pulse, for example, using plethysmographic blood pressure, sonograms or electrocardiograms.

The receptor 6 has means for eliminating extraneous light. For example, the receptor may have an oblong shape similar to, but larger than the shape of the finger. The means for eliminating extraneous light from the receptor is a flexible ring that surrounds an inner surface of an entrance to the receptor. When the finger is inserted, the flexible ring forms a seal around the finger. All surfaces within the device, including surfaces within the receptor, are made non-reflective to minimize stray light.

Fiber optics can be used with the receptor to direct light from the source onto a finger and to collect the dispersed light from said finger for direction through the collimator to the diffraction grating and the detector.

Also, the second derivative calculation or transformation of the said measurement (i.e. of the log of the inverse) may be taken in order to reduce any variation in the result that will be caused by a change in path length for the light. When the device is in use, various users will have fingers of different shape, colour and skin thickness depending on the person being tested, and will produce differing degrees of scattering. The degree of scattering will produce differing effective path lengths for the light in passing through the finger. The effect of the different path lengths and hence differing absorbences can be minimized by taking the second derivative of the measurement. The use of the second derivative calculation minimizes base line shifts due to different path lengths or absorbing water bands, and in addition, enhances the separation of overlapping absorption peaks of different constituents of the mixture being analyzed.

The diffraction grating 12 (which may be a holographic diffraction grating) is used to spatially spread the spectrum of the collected light onto the scanning detector. The scanning linear array detector 15 is preferably a 256 element linear array. The detector collects substantially all of the wavelengths directed onto the finger but only a small portion of the light intensity directed onto the finger.

The noise levels within the device are reduced by the multiple scanning technique whereby the scanner takes a number of measurements and then averages the results. For example, the scanner can scan the entire spectrum of interest two or three hundred times per second between heart beats and then average the results. Depending on the averaging technique used by the scanner and the number of readings taken, a reading can normally be obtained in less than two seconds. The device can be used to determine the concentration of only one component of the sample in the receptor or several components derived from the same averaged spectra. Of course, as the number of components that are being measured increases, the time taken to display and/or print the results increases, due to computer processing time. However, the increased time is still much less than two minutes. The time for taking measurements (at several different wavelengths simultaneously through many repetitions and then averaging the results) is usually approximately 3 minutes and is always less than 5 minutes. The time depends on the number of repetitions and the number of constituents being analyzed. Taking measurements quickly is particularly important when children are the subjects as they often find it very difficult to keep their fingers stationary, even for a brief period of time.

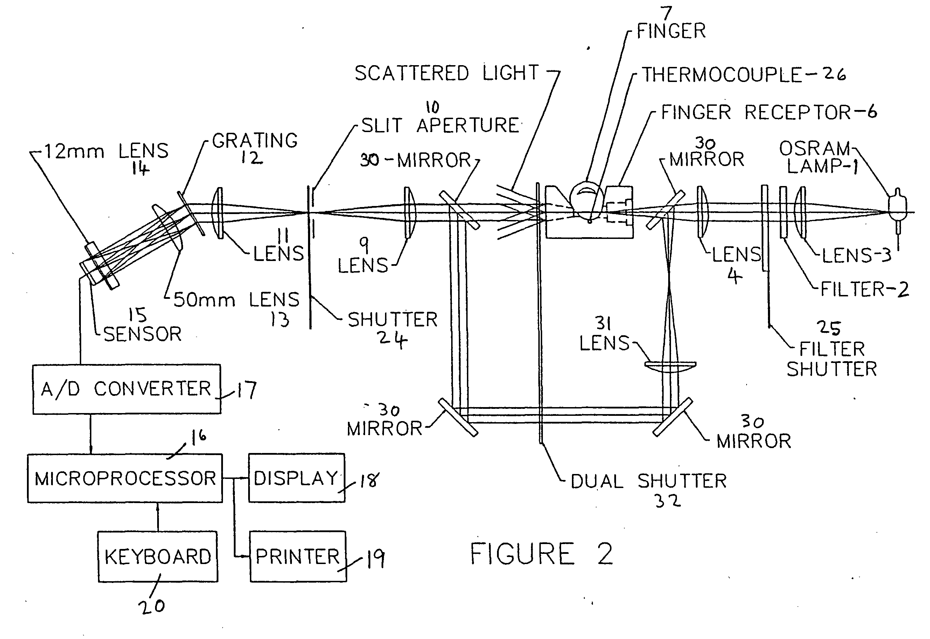

Shown in Figure 2 are the basic elements of a dual beam equivalent of the foregoing instrument, wherein like elements are identified by like reference numerals. There are several important differences in the optical system for the dual beam instrument:

1) A reference beam is directed around the finger receptor 6 through the use of four transmissive, but reflective surfaces 30. The amount of light reflected off the four surfaces is reduced so as to be equivalent to the attenuation provided by the neutral density filter 21 of the single beam instrument of Figure 1.

2) To provide collimated light for the reference beam, an additional lens 31 is inserted to collimate the light which is focused into the finger receptor. This arrangement makes it unnecessary to move the focusing lens 4 as is required with the single beam device.

3) In the dual beam instrument the only moving parts are three shutters: a) a dual shutter 32 to select measurement and reference scans; b) the shutter 24 to block the light during the dark scans; and c) the shutter 25 to insert a calibration filter during wavelength and absorbence calibration.

An advantage of the dual beam instrument is that the reference scan can be taken without removing the finger from the receptor . The operation of the dual beam device is essentially the same as the single beam instrument except that two important advantages accrue from the dual beam arrangement:

1) Continuous measurement is possible;

2) If any drift in instrumentation occurs it can be offset by counterbalancing sequences of measurement and reference scans. For example, a sequence of measurement, reference, reference and measurement scans, when averaged together, would eliminate any linear drift occurring over the time of scanning.

For either instrument, the calibration equation by which the blood glucose was computed was derived from a statistical fitting of the spectral data for a large number of subjects to the actual laboratory measured results for their blood glucose. While a best fit of spectral data is utilized to derive a calibration equation for each constituent desired to be measured, one type of best fit is a least squares best fit. The calibration results were cross-validated using the spectral data for an independent sample of subjects. The particular wavelengths for absorbence were chosen to minimize the standard error of prediction (cross-validation). The equation so generated has the form of:

$$Y = KO + K1(Ai) + K2(Ai).... + Kn(Ai)$$

Where

Y is the concentration of glucose
K0, K1, K2....Kn are constants for n wavelengths
Ai is the measured absorbence or second derivative values of absorbence for ith wavelengths in nanometers (nm) corresponding to the Kn constants.

The constants K0, K1, K2 and Kn are dependent upon the instrumentation including the optics and detectors and so must be determined independently for each instrument design.

The results obtained can vary with the temperature of the finger of the user. Therefore, the device contains a temperature sensor 26 so that the temperature of the finger can be measured rapidly at the time of the spectral sampling. This temperature sensor is typically a small-mass thermocouple. Computer software can then be used to allow the microprocessor to compensate for spectrum deviations due to the temperature. So as not to delay the production of results, the temperature sensor has approximately a 100 millisecond response time.

The scanning detector 15 is a photodiode array that is positioned to intercept, across its length, the dispersed spectrum from the diffraction grating 12. The microprocessor 16 is directed by software to scan the scanning detector and calculate the absorbence spectrum and the second derivative of the spectrum. The microprocessor can then calculate the concentration of the particular components being measured using the absorbence and second derivative values for a number of selected wavelengths. The calibration equation used in each case is determined by the analyte being measured.

The microprocessor can collect up to 100 spectra in a period of one to two seconds and can then immediately calculate the second derivative of the averaged results. Preferably, the results will be digitally displayed for the user. Also, by using the memory capacity of the microprocessor, a user can monitor trends by comparing the most recent result with previous results. For glucose measurements, this will be of great assistance to a diabetic in order to enable the diabetic to determine future insulin dosage requirements.

While examples noted above have been primarily related to measuring glucose concentrations, the device can be used to measure various other components found within human blood or tissue for example, cholesterol, alcohol, lactate, carbon dioxide, nitrogen, cortisol, creatine, creatinine, glycosolated hemoglobin, $Ca^{++}$, Kt $Cl^-$, $HCO_{3-}$, $HPO_{4-}$, ketone bodies, lipids, fat, urea, amino acids, fatty acids and blood oxygenation levels to name a few. Devices for determining glucose levels can be pocket-sized and will be safer, more accurate when used by unskilled persons, less painful and much more convenient than invasive techniques presently being used. The same can be said for devices that measure cholesterol levels. As is well known, alcohol levels are presently measured by either taking a blood sample or a breath sample. Approved breathalyzers are extremely expensive and certain disposable parts must be used for sanitation purposes or are chemically "consumed" as part of the measurement. With the devices embodying the present invention, alcohol levels can be measured quickly and easily without any active participation by the person whose alcohol level is being measured. The devices could even be used when the person being measured was unconscious. Presently, in the field of sports medicine, the level of lactate present in muscles requires a biopsy and blood test. When athletes are in training for an upcoming event, biopsies are impossible because of the damage that they cause to the muscle. With the device embodying the present invention, lactate levels can be determined non-invasively.

Since the second derivative is taken and with the multitude of wavelengths throughout the spectrum being available for analysis, it is possible to distinguish between various components simultaneously. For example, alcohol and glucose, which have presented problems in the prior art, can be distinguished.

While the devices embodying the present invention can be designed to measure one component, it is also possible to design a device to measure several components simultaneously. In fact, a primary application of the device will be for measuring 10 to 40 or more constituents that are now typically measured in a medical or hospital lab after a vial of blood is extracted from the patient. Further, this multi-constituent measurement can be completed and results displayed in seconds right in the doctor's office rather than received in days or weeks as is the practice today.

## Claims

1. A non-invasive device for measuring concentration levels of constituents of blood and tissue in a finger (7) of a living human subject, said device having a polychromatic light source (1), powered by a stabilised power source, that emits a broad spectrum of light in the near infrared range, means (9) for collecting simultaneously substantially all of the wavelengths of said light after said light has been directed onto said finger (7), means (12) for dispersing said collected light into component wavelengths of said collected light, a receptor (6) shaped so that said finger can be placed in contact with said receptor, said receptor having means for eliminating extraneous light, said receptor (6) being located relative to said light source (1) so that when said finger (7) is properly placed in contact with said receptor, said light source can be activated and light from said light source is directed onto said finger, means (15, 16, 17) for taking absorbence measurements from transmitted light from said collected and dispersed light at several wavelengths, means (16, 17) for transforming said absorbence measurements to enhance detection of at least one constituent from other constituents by using a calibration equation for said at least one constituent, and means (16, 17) for determining the concentration level of said at least one constituent of said blood and then producing a result for each concentration level determined, wherein said receptor (6) has an entrance (5) and an exit (8) for light from said light source (1), located generally opposite one another, with a constant distance therebetween, in the optical path between said light source (1) and collecting means (9) so that said finger, when properly placed in the receptor (6) between the entrance and exit, will contact the receptor adjacent both the entrance and exit with the length of the optical path through said finger corresponding to the distance between said entrance and exit, a filter (2) is provided through which said light from said light source (1) passes to limit the light directed onto said finger through said entrance to a range of wavelengths from approximately 650 nm to approximately 1250 nm, said dispersing means (12) is operable to disperse said collected light into a dispersed spectrum comprising the component wavelengths of the collected light, and said means (15, 16, 17) for taking absorbence measurements is operable to take absorbence measurements at several different wavelengths simultaneously over said dispersed spectrum.

2. A device as claimed in claim 1, wherein the calibration equation is derived from a best fit of spectral data obtained by said device to actual measured levels obtained coincidentally.

3. A device as claimed in claim 2, wherein the means (16, 17) for transforming said measurements to enhance detection of at least one constituent from other constituents is operable to take the log of the inverse of the transmittance of said collected light and further to take the second derivative of said log of the inverse.

4. A device as claimed in claim 2 or 3, wherein the means for taking absorbence measurements includes a linear array detector (15) having an analog to digital converter (17) connected thereto, said detector accumulating a charge with time, said charge being measured by an output to the converter, said means for measuring including means (16) for taking measurements when said charge has accumulated to a predetermined range, said range being close to a maximum value for the analog to digital converter (17).

5. A device as claimed in any one of claims 1, 3 or 4, wherein the filter (3) limits the light to a range of approximately 700 nm to approximately 1100 nm.

6. A device as claimed in any one of claims 1, 3 or 4, wherein the means for taking absorbence measurements is a linear array detector (15) and a microprocessor (16), said linear array detector receiving said dispersed light after the dispersed light has been directed onto said finger and collected, said microprocessor causing said linear array detector to be scanned, said detector being connected to said microprocessor for taking said measurements.

7. A device as claimed in any one of claims 1, 3 or 4, wherein the dispersing means (12) is a grating.

8. A device as claimed in any one of claims 1, 3 or 4, wherein there are means for detecting a pulse within said finger placed in contact with said receptor (6), there being means (16) to control said device to use measurements taken immediately subsequent to the detection of a pulse and prior to a next pulse so that all measurements upon which a result is based are taken between pulses.

9. A device as claimed in any one of claims 1, 3 or 4, wherein there are means for detecting a pulse within said finger placed in contact with said receptor (6), there being means (16) to control said device to use measurements taken during a pulse so that all measurements upon which a result is based are taken during a pulse.

10. A device as claimed in claim 2, wherein the device has a temperature sensor (26) for measuring the temperature at said finger (7) located near the receptor with means for adjusting the measurements taken based on variations in said temperature.

11. A device as claimed in claim 4, wherein the linear array detector (15) has 256 elements.

12. A device as claimed in any one of claims 1, 3 or 4, wherein the collecting means are lenses (9, 11).

13. A device as claimed in any one of claims 1, 3 or 4, wherein there are means for detecting a pulse based on monitoring plethysmographic blood pressure.

14. A device as claimed in any one of claims 1, 3 or 4, wherein there are means for detecting a pulse based on a sonogram.

15. A device as claimed in any one of claims 1, 3 or 4, wherein there are means for detecting a pulse based on an electrocardiogram.

16. A device as claimed in any one of claims 1, 3 or 4, wherein there is a collimator (3) located between the polychromatic light source (1) and said receptor (6), so that light from the polychromatic light source passes through said collimator before passing into said receptor.

17. A device as claimed in any one of claims 1, 3 or 4, wherein the collecting means is a lens (9), said lens being oriented and shaped to focus said light on a slit (10), said light passing through said slit to a second lens (11) to collimate the light onto a diffraction grating (12), the means for taking absorbence measurements being a linear array detector (15), said detector producing an output that is passed to a microprocessor (16) which takes the measurements and is controlled by computer software to transform said measurements and determine the concentration level of at least one component of said mixture and produce a result.

18. A device as claimed in claim 4, wherein the measurements are taken when the charge is approximately 73% to approximately 85% of the maximum value for said converter (17).

19. A device as claimed in any one of claims 1, 2 or 3, wherein the means for taking absorbence measurements is a linear array detector (15) and a microprocessor (16), and there are means for reducing noise levels within said device by a scanning technique whereby the linear array detector scans the entire spectrum of interest many times per second for several repetitions ranging from approximately 8 to approximately 64 repetitions and the microprocessor then averages the results.

20. A device as claimed in any one of claims 1, 3 or 4, wherein fiber optics is used to direct said light onto said finger (7) and to collect the light after said light has been directed onto said finger.

21. A device as claimed in any one of claims 1, 3 or 4, wherein the collecting means are reflective surfaces.

22. A device as claimed in any one of claims 1, 3 or 4, wherein the dispersing means is a holographic diffraction grating.

23. A device as claimed in any one of claims 1, 3 or 4, wherein the receptor (6) has dual beams so that reference measurements can be taken while the finger (7) remains in contact with the receptor.

24. A device as claimed in any one of claims 1, 3 or 4, wherein the concentration level of constituents that are measured are selected from, but not limited to, the group of: amino acid, nitrogen, blood oxygenation, carbon dioxide, cortisol, creatine, creatinine, glucose, ketone bodies, lipids, fat, urea, amino acids, fatty acids, glycosolated hemoglobin, cholesterol, alcohol, lactate, $Ca^{++}$, $K^+$, $Cl^-$, $HCO_3^-$, and $HPO_4^-$.

25. A device as claimed in claim 23, wherein the means (15) for taking absorbence measurements is a linear array detector comprising a photo diode array, and the dispersing means (12) is a grating, the light being collected from the grating by the photo diode array that is positioned to intercept the dispersed spectrum of light across its length.

26. A device as claimed in claim 10, wherein the temperature sensor (26) is a thermocouple designed with a fast response time, of approximately 100 milliseconds, and there is a microprocessor (16) to compensate for spectrum deviations due to temperature.

27. A device as claimed in any one of claims 1, 3 or 4, wherein the means (15) for taking absorbence measurements is a linear array detector and a microprocessor, and the measurement of several wavelengths simultaneously, together with a speed of the microprocessor, result in the time for taking a measurement and receiving a result ranging from three to five minutes depending on the number of constituents being analyzed.

28. A device as claimed in any one of claims 1, 3 or 4, wherein there are means (16) for transforming said measurements over said dispersed spectrum to enhance detection of at least two constituents by using a calibration equation for each of said at least two constituents.

29. A non-invasive method for measuring concentration levels of blood and tissue constituents within a finger (7) of a living human subject, using a polychromatic light source (1) that emits a broad spectrum of light in the near infrared range, which comprises locating the finger (7) in contact with a receptor (6), directing said light onto said finger, collecting simultaneously substantially all of the wavelengths of said light after said light has been directed onto said finger (7), collimating the collected light, dispersing the collimated light into component wavelengths of said collected light onto a linear array detector (15), taking absorbence measurements with said linear array detector of transmitted light from said dispersed light at several different wavelengths, scanning said linear array detector and passing measurements from transmitted light from said dispersed light at several different wavelengths simultaneously to a microprocessor (16), taking a reference set of measurements, transforming said measurements to enhance the detection of at least one constituent from other constituents by using a calibration equation for said at least one constituent, and determining the concentration level of said at least one constituent of said blood and tissue and producing a result for each concentration level determined, the method including locating said finger (7) in contact with the receptor (6) adjacent an entrance (5) and an exit (8) for light in said receptor (6), said entrance and exit being located generally opposite each other with a constant distance therebetween in the optical path between said light source (1) and linear array detector (15) so that the length of the optical path through said finger corresponds to the distance between said entrance and exit, directing said light from said light source (1), through a filter (2) which limits the light directed onto said finger to a range of wavelengths from approximately 650 nm to approximately 1250 nm, onto said finger through said entrance (5), dispersing said collimated light from said exit into a dispersed spectrum comprising the component wavelengths of the collected light onto said linear array detector (15), and taking absorbence measurements from transmitted light from said dispersed light at several different wavelengths simultaneously over said dispersed spectrum.

30. A method as claimed in claim 29, wherein the measurements are transformed to enhance the detection of at least one constituent from other constituents by taking the log of the inverse of the transmittance and further by calculating the second derivative of said log of said inverse.

31. A method as claimed in claim 29, wherein there is an analog to digital converter (17) connected to said detector (15), said detector accumulating a charge with time, said charge being measured by an output to the converter, said method including the steps of making a calculation to determine when the charge will be within a predetermined range and taking measurements when said charge has accumulated to said predetermined range within approximately 75% of a maximum charge for said detector.

32. A method as claimed in any one of claims 29 to 31, including the step of deriving the calibration equation from a best fit of spectral data obtained using the device to actual measured levels obtained coincidentally.

33. A method as claimed in claim 30, wherein there is an analog to digital converter (17) connected to said detector (15), said detector accumulating a charge with time, said charge being measured by an output to the converter, said method including the step of activating said means for taking measurements when said charge has accumulated to a predetermined range within 73% of a maximum value for said converter.

34. A method as claimed in claim 33, including the step of controlling the charge of the detector so that the accumulated charge is within approximately 73% to approximately 85% of the maximum value for said converter when a measurement is taken.

35. A method as claimed in claim 29, including the step of passing said light from the light source through the filter (3) to limit the light to a range from approximately 700 nm to approximately 1100 nm.

36. A method as claimed in any one of claims 29 to 31, including the steps of scanning the detector (15) using the microprocessor (16), taking several measurements in rapid succession, and averaging the results with the microprocessor.

37. A method as claimed in any one of claims 29 to 31, which includes means for detecting a pulse within said finger (7), and taking measurements between pulses.

38. A method as claimed in any one of claims 29 to 31, which includes detecting a pulse within said finger (7), and taking measurements during a pulse.

39. A method as claimed in any one of claims 29 to 31, which includes detecting a pulse within said finger (7) with said microprocessor (16), and controlling the microprocessor (16) to accept only those measurements taken at the same time relative to said pulse.

40. A method as claimed in any one of claims 29 to 31, including the steps of controlling the microprocessor (16) to scan the entire spectrum of interest many times per second for several repetitions ranging from approximately 8 to approximately 64 repetitions and having the microprocessor average the results.

41. A method as claimed in any one of claims 29 to 31, including the step of measuring the concentration level of constituents that are selected from but not limited to the group of: amino acid, nitrogen, blood oxygenation, carbon dioxide, cortisol, creatine, creatinine, glucose, ketone bodies, lipids, fat, urea, amino acids, fatty acids, glycosolated hemoglobin, cholesterol, alcohol, lactate, $Ca^{++}$, $K^+$, $Cl^-$, $HCO_3^-$, and $HPO_4^-$.

**Patentansprüche**

1. Eine nichtinvasive Vorrichtung für die Messung der Konzentrationsspiegel der wesentlichen Bestandteile von Blut und Gewebe in einem Finger (7) eines lebenden, menschlichen Subjektes, wobei besagte Vorrichtung eine polychromatische Lichtquelle (1) aufweist, energieversorgt durch eine stabilisierte Energiequelle, welche ein breites Spektrum von Licht in der Nähe des Infrarotbereiches emitiert, Mittel (9) für das simultane Sammeln im wesentlichen aller Wellenlängen von besagtem Licht, nachdem besagtes Licht auf besagten Finger (7) gerichtet wurde, Mittel (12) für die Aufsplittung von besagtem, gesammeltem Licht in die Komponenten der Wellenlängen von besagtem, gesammeltem Licht, einen Rezeptor (6), welcher so geformt ist, dass besagter Finger in Kontakt mit besagtem Rezeptor gebracht werden kann, wobei besagter Rezeptor Mittel für die Eliminierung von systemfremden Licht ausgestattet ist, wobei besagter Rezeptor (6) relativ zu besagter Lichtquelle (1) angeordnet ist, so dass, wenn besagter Finger (7) richtig in Kontakt mit besagtem Rezeptor plaziert ist, besagte Lichtquelle aktiviert werden kann und Licht von besagter Lichtquelle auf besagten Finger geleitet wird, Mittel (15, 16, 17) für die Aufnahme der absorbierten Messungen von dem gesendeten Licht von besagtem gesammelten und in mehrere Wellenlängen aufgesplittetem Licht, Mittel (16, 17) für die Transformierung besagter absorbierter Messungen um die Detektierung von wenigstens einem wesentlichen Bestandteil unter anderen wesentlichen Bestandteilen anzuheben, durch die Anwendung einer Kalibrierungsgleichung für besagten, wenigstens einen wesentlichen Bestandteil, und Mittel (16, 17) für die Bestimmung des Konzentrationsspiegels von besagtem, wenigstens einen wesentlichen Bestandteil von besagtem Blut, und dann die Erzeugung eines Ergebnisses für jeden bestimmten Konzentrationsspiegel, wobei besagter Rezeptor (6) einen Eingang (5) und einen Ausgang (8) für das Licht von besagter Lichtquelle (1) aufweist, im wesentlichen sich gegenüberliegend angeordnet, mit einem konstanten Abstand in dem optischen Pfad zwischen besagter Lichtquelle (1) und besagten Sammelmitteln (9), so dass besagter Finger, wenn dieser richtig in dem Rezeptor (6) zwischen dem Eingang und dem Ausgang plaziert ist, dieser den Rezeptor unmittelbar berührt, sowohl neben dem Eingang als auch dem Ausgang mit der Länge des optischen Pfades durch besagten Finger, korrespondierend mit dem Abstand zwischen besagtem Eingang und besagtem Ausgang, ein Filter (2) zur Verfügung gestellt ist, durch welches besagtes Licht von besagter Lichtquelle (1) hindurchgeht, um das Licht zu begrenzen, welches auf besagten Finger durch besagten Eingang geleitet wird, in einem Bereich der Wellenlänge von etwa 650 nm bis etwa 1250 nm, wobei besagtes Mittel (12) zur Aufsplittung dazu verwendbar ist, besagtes gesammeltes Licht in ein aufgesplittetes Spektrum aufzusplitten, welche die Wellenlängenkomponenten des gesammelten Lichtes enthält, und besagte Mittel (15, 16, 17) für die Aufnahme der absorbierten Messungen dazu geeignet sind, Absorbtionsmessungen simultan bei mehreren, unterschiedlichen Wellenlängen über besagtes aufgesplittetes Spektrum durchzuführen.

2. Eine Vorrichtung wie beansprucht in Anspruch 1, wobei die Kalibrierungsgleichung von einem besten Ergebnis spektraler Daten hergeleitet wird, welche durch aktuell gemessene Werte erreicht werden, die übereinstimmend erzielbar sind.

3. Eine Vorrichtung wie beansprucht in Anspruch 2, wobei die Mittel (16, 17) für die Transformierung besagter Messergebnisse zur Erhöhung der Detektion von wenigstens einem wesentlichen Bestandteil aus anderen wesentlichen Bestandteilen dazu geeignet sind, den log der Inversen der Übermittlung von besagtem, gesammeltem Licht anzuheben, und weiters eine zweite Ableitung von besagtem log der Inversen zu nehmen.

4. Eine Vorrichtung wie beansprucht in den Ansprüchen 2 oder 3, wobei die Mittel für die Aufnahme der absorbierten Messungen einen linearen Zeilendetektor (15) beinhalten, welcher einen A/D Konverter (17) aufweist, welcher damit verbunden ist, wobei besagter Detektor eine zeitliche Belastung akkumuliert, wobei besagte Belastung gemessen wird an dem Ausgang des Konverters, besagte Mittel für die Messung beinhalten Mittel (16) für die Erstellung der Messung, wenn besagte Belastung einen vorherbestimmten Bereich akkumuliert hat, wobei besagter Bereich nahe dem maximalen Wert für den A/D Konverter (17) liegt.

5. Eine Vorrichtung wie beansprucht in einem der vorangehenden Ansprüche 1, 3 oder 4, wobei das Filter (3) das Licht in einem Bereich von etwa 700 nm bis etwa 1100 nm eingrenzt.

6. Eine Vorrichtung wie beansprucht in einem der vorangehenden Ansprüche 1, 3 oder 4, wobei die Mittel für die Aufnahme der absorbierten Messungen ein linearer Zeilendetektor (15) und ein Mikroprozessor (16) sind, wobei besagter linearer Zeilendetektor besagtes, aufgesplittetes Licht empfängt, nachdem das aufgesplittete Licht auf besagten Finger gerichtet und gesammelt wurde, besagter Mikroprozessor besagten linearer Zeilendetektor veranlasst zu scannen, wobei besagter Detektor für die Aufnahme der Messung mit dem Mikroprozessor verbunden ist.

7. Eine Vorrichtung wie beansprucht in einem der Ansprüche 1, 3 oder 4, wobei das Mittel zur Aufsplittung (12) ein Beugungsgitter ist.

8. Eine Vorrichtung wie beansprucht in einem der Ansprüche 1, 3 oder 4, wobei Mittel vorhanden sind für die Detektierung eines Pulses innerhalb besagtem Finger, welcher in Kontakt mit besagtem Rezeptor (6) ist, wobei Mittel (16) für die Kontrolle von besagter Vorrichtung für die Verwendung der Messungen, welche unmittelbar nach der Detektierung des Pulses und vor dem nächsten Puls aufgenommen werden, vorhanden sind, so dass alle Messungen über welche ein Ergebnis erstellt wird, zwischen den Pulsen aufgenommen werden.

9. Eine Vorrichtung wie beansprucht in einer der Ansprüche 1, 3 oder 4, wobei Mittel vorhanden sind für die Detektierung eines Pulses in besagtem Finger, welcher in Kontakt mit besagtem Rezeptor (6) gebracht ist, wobei Mittel (16) zur Kontrolle besagter Vorrichtung zur Verwendung der Messungen, welche während eines Pulses durchgeführt werden, vorhanden sind, so dass alle Messungen auf deren Basis ein Ergebnis hergeleitet wird während einem Puls aufgenommen werden.

10. Eine Vorrichtung wie beansprucht in Anspruch 2, wobei die Vorrichtung einen Temperatursensor (26) aufweist, für die Messung der Temperatur an besagtem Finger (7), angeordnet nahe dem Rezeptor, mit Mitteln für die Einstellung der Messungen welche basierend auf Variationen in besagter Temperatur durchgeführt werden.

11. Eine Vorrichtung wie beansprucht in Anspruch 4, wobei der lineare Zeilendetektor (15) 256 Elemente aufweist.

12. Eine Vorrichtung wie beansprucht in einem der Ansprüche 1, 3 oder 4, wobei die Sammelmittel Linsen (9, 11) sind.

13. Eine Vorrichtung wie beansprucht in einem der Ansprüche 1, 3 oder 4, wobei Mittel für die Ermittlung des Blutdruckes anhand einer plethysmographischen Anzeige vorhanden sind.

14. Eine Vorrichtung wie beansprucht in einem der Ansprüche 1, 3 oder 4, wobei Mittel vorhanden sind für die Bestimmung des Pulses basierend auf einem Sonogramm.

15. Eine Vorrichtung wie beansprucht in einem der Ansprüche 1, 3 oder 4, wobei Mittel vorhanden sind für die Bestimmung des Pulses basierend auf einem Elektrokardiogramm.

16. Eine Vorrichtung wie beansprucht in einem der Ansprüche 1. 3 oder 4, wobei ein Kollimator (3) vorhanden ist, angeordnet zwischen der polychromatischen Lichtquelle (1) und besagtem Rezeptor (6), so dass das Licht von der polychromatischen Lichtquelle durch gesagten Kollimator hindurchgeht, bevor sie in den Rezeptor hineingeht.

17. Eine Vorrichtung wie beansprucht in einem der Ansprüche 1, 3 oder 4, wobei das Sammelmittel eine Linse (9) ist, besagte Linse ausgerichtet und geformt ist, um besagtes Licht an einem Schlitz (10) zu fokussieren, besagtes Licht durch den Schlitz hindurchgehend zu einer zweiten Linse (11), um das Licht auf einem Diffraktionsgitter (12) zu kollimieren, wobei das Mittel für die Aufnahme der absorbierten Messungen ein linearer Zeilendetektor (15) ist, wobei besagter Detektor einen Ausgang produziert, welcher an den Mikroprozessor (16) gesendet wird, welcher die Messungen übernimmt, und kontrolliert wird durch eine Computersoftware, um besagte Messungen zu transformieren und den Konzentrationsspiegel von wenigstens einer Komponente von besagter Mixtur zu bestimmen und ein Ergebnis zu ermitteln.

18. Eine Vorrichtung wie beansprucht in Anspruch 4, wobei die Messungen aufgenommen werden, wenn die Belastung in etwa 73 % bis in etwa 85 % des maximalen Wertes von besagtem Konverter (17) betragen.

19. Eine Vorrichtung wie beansprucht in einem der Ansprüche 1, 2 oder 3, wobei die Mittel für die Aufnahme der absorbierten Messungen ein linearer Zeilendetektor (15) und ein Mikroprozessor (16) sind, und wobei Mittel für die Reduzierung des Rauschpegels innerhalb besagter Vorrichtung durch einen Scantechnik vorhanden sind, wobei der lineare Zeilendetektor das gesamte interessierende Spektrum mehrere Male pro Sekunde für mehrere Wiederholungen scant, welche sich in einem Bereich von etwa 8 bis etwa 64 Wiederholungen erstrecken, und wonach dann der Mikroprozessor einen durchschnittlichen Ergebniswert ermittelt.

20. Eine Vorrichtung wie beansprucht in einem der Ansprüche 1, 3 oder 4, wobei Glasfaseroptik benutzt wird, um besagtes Licht auf besagten Finger (7) zu lenken und das Licht zu sammeln, nachdem besagtes Licht auf besagten Finger gelenkt wurde.

21. Eine Vorrichtung wie beansprucht in einem der Ansprüche 1, 3 oder 4, wobei die Sammelmittel reflektierende Oberflächen sind.

22. Eine Vorrichtung wie beansprucht in einem der Ansprüche 1, 3 oder 4, wobei das Aufsplittungsmittel ein holographisches Diffraktionsgitter ist.

23. Eine Vorrichtung wie beansprucht in einem der Ansprüche 1, 3 oder 4, wobei der Rezeptor (6) einen dualen Strahl aufweist, so dass Referenzmessungen durchgeführt werden können, während der Finger (7) in Kontakt mit dem Rezeptor verbleibt.

24. Eine Vorrichtung wie beansprucht in einem der Ansprüche 1, 3 oder 4, der Konzentrationsspiegel der wesentlichen Bestandteile die gemessen werden selektiert werden von, jedoch nicht begrenzt werden auf die Gruppe von: Aminosäuren, Stickstoff, Blutoxidierungen, Kohlendioxyde, Kortisol, Creatine, Creatinine, Glukose, ketone Körper, Lipide, Fett, Urea, Aminosäuren, Fettsäuren, glykodisierte Hemoglobine, Cholesterol, Alkohol, Laktate, Ca$^{++}$, K$^+$, Cl$^-$, HCO$_3^-$, und HPO$_4^-$.

25. Eine Vorrichtung wie beansprucht in Anspruch 23, wobei das Mittel (15) für die Aufnahme der absorbierten Messungen ein linearer Zeilendetektor ist, beinhaltend eine Fotodiodenzeile, und das Aufsplittungsmittel (12) ein Beugungsgitter ist, wobei das Licht durch das Beugungsgitter der Fotodiodenzeile gesammelt wird, welches positioniert ist, um das entlang seiner Länge aufgesplittette Spektrum abzufangen.

26. Eine Vorrichtung wie beansprucht in Anspruch 10, wobei der Temperatursensor (26) ein thermogekoppeltes Design ist, mit einer schnellen Antwortzeit von etwa 100 Millisekunden ist, und wobei ein Mikroprozessor (16) vorhanden ist, um die temperaturbedingten Spektrumsabweichungen zu kompensieren.

27. Eine Vorrichtung wie beansprucht in einem der Ansprüche 1, 3 oder 4, wobei die Mittel (15) für die Aufnahme der absorbierten Messungen ein linearer Zeilendetektor und ein Mikroprozessor sind, und die Messungen von mehreren Wellenlängen simultan, zusammen mit einer Geschwindigkeit des Mikroprozessors, resultierend in der Zeit für die Aufnahme einer Messung und das Erhalten eines Ergebnisses im Bereich von 3 bis 5 Minuten abhängig von der Anzahl der wesentlichen Bestandteile analysiert werden.

28. Eine Vorrichtung wie beansprucht in einem der Ansprüche 1, 3 oder 4, wobei Mittel (16) vorhanden sind für die Transformierung besagter Messungen, über besagtes, aufgesplittetes Spektrum, um die Detektierung von wenigstens zwei wesentlichen Bestandteilen anzuheben, durch den Einsatz einer Kalibrierungsgleichung für jede der besagten wenigstens zwei wesentlichen Bestandteile.

29. Ein nichtinvasives Verfahren zu Messung des Konzentrationswertes wesentlicher Bestandteile von Blut und Gewebe in einem Finger (7) eines lebenden, menschlichen Subjektes, verwendend eine polychromatische Lichtquelle (1) welche ein breites Spektrum von Licht in der Nähe des infraroten Bereiches emittiert, welche beinhaltet, die Anordnung des Fingers (7) in Kontakt mit einem Rezeptor (6), die Lenkung besagtem Lichtes auf besagtem Finger, simultane Sammlung im wesentlichen aller Wellenlängen von besagtem Licht, nachdem besagtes Licht auf besagten Finger (7) gelenkt wurde, kollimieren des gesammelten Lichtes, Aufsplittung des kollimierten Lichtes in Wellenlängenkomponenten von besagtem, gesammeltem Licht auf einem linearen Zeilendetektor (15), die Aufnahme der absorbiert Messungen mit besagtem, linearen Zeilendetektor von dem gesendeten Licht von besagtem, in mehreren unterschiedlichen Wellenlängen aufgesplittetem Licht, scannen von besagtem, linearem Zeilendetektor, und passieren der Messungen von dem gesendeten Licht, von besagtem in mehrere verschiedene Wellenlängen aufgespaltetem Licht, simultan zu einem Mikroprozessor (16), aufnehmen eines Referenzsatzes von Messungen, transformieren besagter Messungen, um die Detektion von wenigstens einem wesentlichen Bestandteil unter anderen, wesentlichen Bestandteilen, durch die Verwendung einer Kalibrierungsgleichung für besagten, wenigstens einen wichtigen Bestandteil anzuheben, und Bestimmung des Konzentrationswertes von besagtem, wenigstens einen wesentlichen Bestandteil von besagtem Blut und Gewebe, und die Erstellung eines Ergebnisses, für jeden bestimmten Konzentrationswert, wobei das Verfahren beinhaltet, die Anordnung von besagtem Finger (7) in Kontakt mit dem Rezeptor (6), unmittelbar am Eingang (5) und am Ausgang (8) für das Licht in besagtem Rezeptor (6), wobei besagter Eingang und besagter Ausgang im wesentlichen sich gegenüberliegend angeordnet sind, mit einem konstanten Abstand in dem optischen Pfad zwischen besagter Lichtquelle (1) und besagtem linearen Zeilendetektor (15), so dass die Länge des optischen Pfades durch besagten Finger korrespondiert mit dem Abstand zwischen besagtem Eingang und besagtem Ausgang, lenken von besagtem Licht von besagter Lichtquelle (1), durch ein Filter (2), welches Licht beschränkt, welches auf besagten Finger gerichtet wird, auf eine Bandbreite von Wellenlängen von etwa 650 nm bis etwa 1250 nm auf besagten Finger durch besagten Eingang (5), Aufsplittung von besagtem, kollimiertem Licht von besagtem Ausgang in ein aufgesplittetes Spektrum, beinhaltend die Wellenlängenkomponenten des kollimierten Lichtes auf besagtem, linearem Zeilendetektor (15), und Aufnehmen der absorbierten Messungen von dem gesendeten Licht von besagtem, aufgesplitteten Licht an mehreren, unterschiedlichen Wellenlängen simultan über besagtes aufgesplittetes Spektrum.

30. Ein Verfahren wie beansprucht in Anspruch 29, wobei die Messungen transformiert werden um die Bestimmung von wenigstens einem wesentlichen Bestandteil unter anderen wesentlichen Bestandteilen anzuheben, durch das Erfassen des log der Inversen der Lichtdurchlässigkeit und im Weiterem durch die Berechnung der zweiten Ableitung von besagtem log von besagter Inversen.

31. Ein Verfahren wie beansprucht in Anspruch 29, wobei ein A/D Konverter (17) vorhanden ist, welcher mit besagtem Detektor (15) verbunden ist, wobei besagter Detektor die zeitliche Belastung akkumuliert, besagte Belastung gemessen ist an einem Ausgang des Konverters, wobei besagtes Verfahren beinhaltet die Schritte der Durchführung einer Berechnung, um zu bestimmen, wann die Belastung in einem vorher bestimmten Bereich sein wird, und Aufnehmen der Messung, wenn besagte Belastung sich in besagtem, vorher bestimmten Bereich innerhalb etwa 75 % der maximalen Belastung für besagten Detektor akkumuliert hat.

32. Ein Verfahren wie beansprucht in einem der Ansprüche 29 bis 31, beinhaltend die Schritte der Herleitung der Kalibrierungsgleichung von einem besten Ergebnis von spektralen Daten, die erzielt wurden, durch die Verwendung der Vorrichtung für die aktuell gemessenen Werte, welche übereinstimmend erzielt wurden.

33. Ein Verfahren wie beansprucht in Anspruch 30, wobei ein A/D Konverter (17) vorhanden ist, welcher verbunden ist mit besagtem Detektor (15), wobei besagter Detektor eine zeitliche Belastung akkumuliert, besagte Belastung gemessen wird an einem Ausgang des Konverters, besagtes Verfahren beinhaltet die Schritte der Aktivierung besagter Mittel für das Aufnehmen der Messungen, wenn besagte Belastung sich auf einem vorher bestimmten Bereich bis 73 % des maximalen Wertes für besagten Konverter akkumuliert hat.

34. Ein Verfahren wie beansprucht in Anspruch 33, beinhaltend den Schritt der Überwachung der Belastung des Detektors, so dass die akkumulierte Belastung innerhalb eines Bereiches von 73 % bis etwa 85 % des maximalen Wertes für besagten Konverter liegt, wenn die Messung durchgeführt wurde.

35. Ein Verfahren wie beansprucht in Anspruch 29, beinhaltend die Schritte des Passierens von besagtem Licht von besagter Lichtquelle durch das Filter (3), zur Beschränkung des Lichtes auf einen Bereich von etwa 700 nm bis etwa 1100 nm.

36. Ein Verfahren wie beansprucht in einem der Ansprüche 29 bis 31, beinhaltend die Schritte des Scannens des Detektors (15) verwendend den Mikroprozessor (16), Aufnehmen mehrerer Messungen in kurz aufeinander folgenden Abständen, und Mitteln des Ergebnisses mit dem Mikroprozessor.

37. Ein Verfahren wie beansprucht in einem der Ansprüche 29 bis 31, welches beinhaltet Mittel für die Bestimmung eines Pulses in besagtem Finger (7) und Aufnehmen der Messungen zwischen Pulsen.

38. Ein Verfahren wie beansprucht in einem der Ansprüche 29 bis 31, welches beinhaltet, die Ermittlung eines Pulses in besagtem Finger (7) und Aufnehmen der Messungen während einem Puls.

39. Ein Verfahren wie beansprucht in einem der Ansprüche 29 bis 31, welches beinhaltet die Ermittlung eines Pulses in besagtem Finger (7) mit besagtem Mikroprozessor (16) und die Überwachung des Mikroprozessors (16) so dass nur jene Messungen akzeptiert werden, welche in derselben Zeit relativ zu besagtem Puls gemessen werden.

40. Ein Verfahren wie beansprucht in einem der Ansprüche 29 bis 31, beinhaltend den Schritt der Überwachung des Mikroprozessors (16) um das gesamte, interessierende Spektrum mehrere Male pro Sekunde für mehrere Wiederholungen im Bereich von etwa 8 bis etwa 64 Wiederholungen zu scannen, und den Mikroprozessor verwendend um eine durchschnittliches Ergebnis zu berechnen.

41. Ein Verfahren wie beansprucht in einem der Ansprüche 29 bis 31, beinhaltend den Schritt der Messung der Konzentrationswerte der wesentlichen Bestandteile, welche selektiert sind aus, jedoch nicht limitiert sind auf die Gruppe von: Aminosäuren, Stickstoff, Blutoxidierungen, Kohlendioxyde, Kortisol, Creatine, Creatinine, Glukose, ketone Körper, Lipide, Fett, Urea, Aminosäuren, Fettsäuren, glykodisierte Hemoglobine, Cholesterol, Alkohol, Laktate, $Ca^{++}$, $K^+$, $Cl^-$, $HCO_3^-$, und $HPO_4^-$.

## Revendications

1. Dispositif non invasif pour mesurer des niveaux de concentration de constituants de sang et de tissus dans un doigt (7) d'un sujet humain vivant, ledit dispositif ayant une source de lumière polychromatique (1), alimentée par une source d'alimentation stabilisée, qui émet un large spectre de lumière dans le domaine du proche infrarouge, des moyens (9) pour collecter de manière simultanée sensiblement toutes les longueurs d'onde de ladite lumière après que ladite lumière a été dirigée sur ledit doigt (7), des moyens (12) pour disperser ladite lumière collectée en longueurs d'onde des composantes de ladite lumière collectée, un récepteur (6) de forme telle que ledit doigt peut être placé en contact avec ledit récepteur, ledit récepteur ayant des moyens pour éliminer de la lumière étrangère, ledit récepteur (6) étant situé par rapport à ladite source de lumière (1) de sorte que ledit doigt (7) soit placé de manière appropriée en contact avec ledit récepteur, ladite source de lumière peut être activée et la lumière provenant de ladite source de lumière est dirigée sur ledit doigt, des moyens (15, 16, 17) pour prendre des mesures d'absorbance de la lumière transmise à partir de ladite lumière collectée et de ladite lumière dispersée à plusieurs longueurs d'onde, des moyens (16, 17) pour transformer lesdites mesures d'absorbance afin d'améliorer la détection d'au moins un constituant parmi d'autres constituants en utilisant une équation de calibrage pour ledit au moins un constituant, et des moyens (16, 17) pour déterminer le niveau de concentration dudit au moins un constituant dudit sang puis produire un résultat pour chaque niveau de concentration déterminé, dans lequel ledit récepteur (6) possède une entrée (5) et une sortie (8) pour la lumière provenant de ladite source de lumière (1), situées généralement à l'opposé l'une de l'autre, avec une distance constante entre les deux, dans le chemin optique entre ladite source de lumière (1) et des moyens de collecte (9) de sorte que ledit doigt, lorsqu'il est placé de manière appropriée dans le récepteur (6) entre l'entrée et la sortie, va être en contact avec le récepteur qui est adjacent à l'entrée ainsi qu'à la sortie, la longueur du chemin optique à travers ledit doigt correspondant à la distance entre ladite entrée et ladite sortie, un filtre (2) est prévu, à travers lequel passe ladite lumière provenant de ladite source de lumière (1) afin de limiter la lumière dirigée sur ledit doigt à travers ladite entrée à un domaine de longueurs d'onde comprises entre environ 650 nm et environ 1250 nm, lesdits moyens de dispersion (12) pouvant fonctionner de manière à disperser ladite lumière collectée en un spectre dispersé comprenant les longueurs d'onde des composants de la lumière collectée, et lesdits moyens (15, 16, 17) destinés à prendre des mesures d'absorbance peuvent fonctionner pour prendre des mesures d'absorbance à plusieurs longueurs d'onde différentes simultanément sur ledit spectre dispersé.

2. Dispositif selon la revendication 1, dans lequel l'équation de calibrage est dérivée de la meilleure adéquation de données spectrales obtenues par ledit dispositif avec des niveaux mesurés de manière coïncidente.

3. Dispositif selon la revendication 2, dans lequel les moyens (16, 17) pour transformer lesdites mesures afin d'améliorer la détection d'au moins un constituant parmi d'autres constituants peuvent fonctionner pour prendre le log de l'inverse de la transmittance de ladite lumière collectée et pour prendre en outre la dérivée seconde dudit log de l'inverse.

4. Dispositif selon la revendication 2 ou 3, dans lequel les moyens pour prendre des mesures d'absorbance comprennent un détecteur linéaire (15) ayant un convertisseur analogique/numérique (17) connecté à celui-ci, ledit détecteur accumulant une charge avec le temps, ladite charge étant mesurée par une sortie du convertisseur, lesdits moyens pour mesurer comprenant des moyens (16) pour prendre des mesures lorsque ladite charge s'est accumulée jusqu'à une plage prédéterminée, ladite plage étant proche d'une valeur maximale pour le convertisseur analogique/numérique (17).

5. Dispositif selon l'une quelconque des revendications 1, 3 ou 4, dans lequel le filtre (3) limite la lumière à un domaine compris entre environ 700 nm et environ 1100 nm.

6. Dispositif selon l'une quelconque des revendications précédentes 1, 3, ou 4, dans lequel les moyens pour prendre des mesures d'absorbance sont un détecteur linéaire (15) et un microprocesseur (16), ledit détecteur linéaire recevant ladite lumière dispersée après que la lumière dispersée a été dirigée sur ledit doigt et collectée, ledit microprocesseur provoquant le balayage dudit détecteur linéaire, ledit détecteur étant connecté audit microprocesseur pour prendre lesdites mesures.

7. Dispositif selon l'une quelconque des revendications 1, 3 ou 4, dans lequel les moyens de dispersion (12) sont un réseau.

8. Dispositif selon l'une quelconque des revendications 1, 3 ou 4, dans lequel sont disposés des moyens pour détecter une pulsation à l'intérieur dudit doigt qui est placé en contact avec ledit récepteur (6), des moyens (16) étant prévus pour contrôler ledit dispositif afin d'utiliser des mesures prises immédiatement après la détection d'une pulsation et avant une pulsation suivante de sorte que toutes les mesures sur lesquelles est basé un résultat soient prises entre des pulsations.

9. Dispositif selon l'une quelconque des revendications 1, 3 ou 4, dans lequel sont disposés des moyens pour détecter une pulsation à l'intérieur dudit doigt qui est placé en contact avec ledit récepteur (6), des moyens (16) étant prévus pour contrôler ledit dispositif afin d'utiliser des mesures prises durant une pulsation de sorte que toutes les mesures sur lesquelles est basé un résultat soient prises durant une pulsation.

10. Dispositif selon la revendication 2, dans lequel le dispositif possède un détecteur de température (26) pour détecter la température au niveau dudit doigt (7) qui est situé à proximité du récepteur avec des moyens pour ajuster les mesures prises en se basant sur des variations de ladite température.

11. Dispositif selon la revendication 4, dans lequel le détecteur linéaire (15) possède 256 éléments.

12. Dispositif selon l'une quelconque des revendications 1, 3, ou 4, dans lequel les moyens de collecte sont des lentilles (9, 11).

13. Dispositif selon l'une quelconque des revendications 1, 3, ou 4, dans lequel sont disposés des moyens pour détecter une pulsation en se basant sur le contrôle de la pression sanguine pléthysmographique.

14. Dispositif selon l'une quelconque des revendications 1, 3 ou 4, dans lequel sont disposés des moyens pour détecter une pulsation en se basant sur un sonogramme.

15. Dispositif selon l'une quelconque des revendications 1, 3 ou 4, dans lequel sont disposés des moyens pour détecter une pulsation en se basant sur un électrocardiogramme.

16. Dispositif selon l'une quelconque des revendications 1, 3 ou 4, dans lequel se trouve un collimateur (3) situé entre la source de lumière polychromatique (1) et ledit récepteur (6), de sorte que la lumière provenant de la source de lumière polychromatique passe à travers ledit collimateur avant de passer dans ledit récepteur.

17. Dispositif selon l'une quelconque des revendications 1, 3 ou 4, dans lequel les moyens de collecte sont une lentille (9), ladite lentille étant orientée et ayant une forme appropriée pour focaliser ladite lumière sur une fente (10), ladite lumière passant à travers ladite fente vers une seconde lentille (11) pour collimater la lumière sur un réseau de diffraction (12), les moyens pour prendre des mesures d'absorbance étant un détecteur linéaire (15), ledit détecteur produisant une sortie qui est acheminée vers le microprocesseur (16) qui prend les mesures et qui est contrôlé par un logiciel informatique afin de transformer lesdites mesures et de déterminer le niveau de concentration d'au moins un composant dudit mélange et produire un résultat.

18. Dispositif selon la revendication 4, dans lequel les mesures sont prises lorsque la charge est dans un pourcentage d'environ 73 % à environ 85 % par rapport à la valeur maximale pour ledit convertisseur (17).

19. Dispositif selon l'une quelconque des revendications 1, 2 ou 3, dans lequel les moyens pour prendre des mesures d'absorbance sont un détecteur linéaire (15) et un microprocesseur (16), et dans lequel sont prévus des moyens pour réduire les niveaux de bruit à l'intérieur dudit dispositif par une technique de balayage, moyennant quoi le détecteur linéaire balaye le spectre concerné en entier plusieurs fois par seconde à plusieurs reprises comprises dans la plage allant d'environ 8 à environ 64 reprises, le microprocesseur réalisant ensuite une moyenne des résultats.

20. Dispositif selon l'une quelconque des revendications 1, 3 ou 4, dans lequel des fibres optiques sont utilisées pour diriger ladite lumière sur ledit doigt (7) et pour collecter la lumière après que ladite lumière a été dirigée sur ledit doigt.

21. Dispositif selon l'une quelconque des revendications 1, 3 ou 4, dans lequel les moyens de collecte sont des surfaces réfléchissantes.

22. Dispositif selon l'une quelconque des revendications 1, 3 ou 4, dans lequel les moyens de dispersion est un réseau de diffraction holographique.

23. Dispositif selon l'une quelconque des revendications 1, 3 ou 4, dans lequel le récepteur (6) possède deux faisceaux de sorte que les mesures de référence puissent être prises tandis que le doigt (7) reste en contact avec le récepteur.

24. Dispositif selon l'une quelconque des revendications 1, 3 ou 4, dans lequel est mesuré le niveau de concentration des constituants qui sont sélectionnés, sans s'y limiter, dans le groupe constitué par : un acide aminé, l'azote, l'oxygénation sanguine, le dioxyde de carbone, le cortisol, la créatine, la créatinine, le glucose, les corps cétoniques, les lipides, les graisses, l'urée, les acides aminés, les acides gras, l'hémoglobine glycosée, le cholestérol, l'alcool, le lactate, $Ca^{++}$, $K^+$, $Cl^-$, $HCO_3^-$, et $HPO_4^-$.

**25.** Dispositif selon la revendication 23, dans lequel les moyens (15) pour prendre des mesures d'absorbance sont un détecteur linéaire comprenant un ensemble de photodiodes, et dans lequel les moyens de dispersion (12) sont un réseau, la lumière étant collectée à partir du réseau par l'ensemble de photodiodes qui est positionné de manière à intercepter le spectre dispersé de lumière à travers sa longueur.

**26.** Dispositif selon la revendication 10, dans lequel le détecteur de température (26) est un thermocouple conçu avec un temps de réponse rapide, d'environ 100 millisecondes, et dans lequel est prévu un microprocesseur (16) destiné à compenser des déviations spectrales en raison de la température.

**27.** Dispositif selon l'une quelconque des revendications 1, 3 ou 4, dans lequel les moyens (15) pour prendre des mesures d'absorbance est un détecteur linéaire et un microprocesseur, et dans lequel la mesure de plusieurs longueurs d'onde simultanément, conjointement avec une vitesse du microprocesseur, aboutissent au temps pour prendre une mesure et recevoir un résultat compris entre trois et cinq minutes en fonction du nombre de constituants qui est analysé.

**28.** Dispositif selon l'une quelconque des revendications 1, 3 ou 4, dans lequel sont prévus des moyens (16) pour transformer lesdites mesures sur ledit spectre dispersé pour améliorer la détection d'au moins deux constituants en utilisant une équation de calibrage pour chacun desdits au moins deux constituants.

**29.** Procédé non invasif pour mesurer des niveau de concentration de constituants de sang et de tissus à l'intérieur d'un doigt (7) d'un sujet humain vivant, utilisant une source de lumière polychromatique (1) qui émet un large spectre de lumière dans le domaine du proche infrarouge, ledit dispositif comprenant les étapes consistant à mettre le doigt (7) en contact avec un récepteur (6), diriger ladite lumière sur ledit doigt, collecter de manière simultanée sensiblement toutes les longueurs d'onde de ladite lumière après que ladite lumière a été dirigée sur ledit doigt (7), collimater la lumière collectée, disperser la lumière collimatée en longueurs d'onde des composantes de ladite lumière collectée sur un détecteur linéaire (15), prendre des mesures d'absorbance avec ledit détecteur linéaire de lumière transmise à partir de ladite lumière dispersée à plusieurs longueurs d'onde différentes, balayer ledit détecteur linéaire et faire passer des mesures de la lumière transmise à partir de ladite lumière dispersée à plusieurs longueurs d'onde différentes simultanément vers un microprocesseur (16), prendre un ensemble de mesures de référence, transformer lesdites mesures pour améliorer la détection d'au moins un constituant parmi d'autres constituants en utilisant une équation de calibrage pour ledit au moins un constituant, et déterminer le niveau de concentration dudit au moins un constituant dudit sang et desdits tissus et produire un résultat pour chaque niveau de concentration déterminé, le procédé comprenant les étapes consistant à mettre ledit doigt (7) en contact avec le récepteur (6) qui est adjacent à une entrée (5) et à une sortie (8) pour la lumière dans ledit récepteur (6), ladite entrée et ladite sortie étant situées généralement de manière opposée l'une par rapport à l'autre, avec une certaine distance entre les deux dans le chemin optique entre ladite source de lumière (1) et le détecteur linéaire (15) de sorte que la longueur du chemin optique à travers ledit doigt corresponde à la distance entre ladite entrée et ladite sortie, diriger ladite lumière à partir de ladite source de lumière (1) à travers un filtre (2) qui limite la lumière dirigée sur ledit doigt à un domaine de longueurs d'onde comprises entre environ 650 nm et environ 1250 nm, sur ledit doigt à travers ladite entrée (5), disperser ladite lumière collimatée à partir de ladite sortie dans un spectre dispersé comprenant les longueurs d'onde des composants de la lumière collectée sur ledit détecteur linéaire (15), et prendre des mesures d'absorbance à partir de la lumière transmise à plusieurs longueurs d'onde différentes de manière simultanée sur ledit spectre dispersé.

**30.** Procédé selon la revendication 29, dans lequel les mesures sont transformées pour améliorer la détection d'au moins un constituant parmi d'autres constituants en prenant le log de l'inverse de la transmittance et en calculant en outre la dérivée seconde dudit log de ladite inverse.

**31.** Procédé selon la revendication 29, dans lequel est prévu un convertisseur analogique/numérique (17) qui est connecté audit détecteur (15), ledit détecteur accumulant une charge avec le temps, ladite charge étant mesurée par une sortie vers le convertisseur, ledit procédé comprenant les étapes consistant à réaliser un calcul pour déterminer le moment où la charge va être comprise dans une plage prédéterminée et à prendre des mesures lorsque ladite charge s'est accumulée jusqu'à ladite plage prédéterminée dans un pourcentage d'environ 75 % par rapport à une charge maximale pour ledit détecteur.

**32.** Procédé selon l'une quelconque des revendications 29 à 31, comprenant l'étape consistant à dériver l'équation de calibrage à partir d'une adéquation optimale de données spectrales obtenues en utilisant le dispositif avec des niveaux mesurés réels obtenus de manière coïncidente.

33. Procédé selon la revendication 30, dans lequel est prévu un convertisseur analogique/numérique (17) qui est connecté audit détecteur (15), ledit détecteur accumulant une charge avec le temps, ladite charge étant mesurée par une sortie vers le convertisseur, ledit procédé comprenant l'étape consistant à activer lesdits moyens pour prendre des mesures lorsque ladite charge s'est accumulée jusqu'à une plage prédéterminée dans un pourcentage de 73 % par rapport à une valeur maximale pour ledit convertisseur.

34. Procédé selon la revendication 33, comprenant l'étape consistant à contrôler la charge du détecteur de sorte que la charge accumulée soit dans un pourcentage d'environ 73 % à 85 % par rapport à la valeur maximale dudit convertisseur lorsqu'une mesure est prise.

35. Procédé selon la revendication 29, comprenant l'étape consistant à faire passer ladite lumière provenant de la source de lumière à travers le filtre (3) afin de limiter la lumière à une plage allant d'environ 700 nm à environ 1100 nm.

36. Procédé selon l'une quelconque des revendications 29 à 31, comprenant les étapes consistant à balayer le détecteur (15) en utilisant le microprocesseur (16), prendre plusieurs mesures successives rapidement et faire une moyenne des résultats avec le microprocesseur.

37. Procédé selon l'une quelconque des revendications 29 à 31, comprenant des moyens pour détecter une pulsation à l'intérieur dudit doigt (7) et prendre des mesures entre des pulsations.

38. Procédé selon l'une quelconque des revendications 29 à 31, comprenant l'étape consistant à détecter une pulsation à l'intérieur dudit doigt (7) et prendre des mesures durant une pulsation.

39. Procédé selon l'une quelconque des revendications 29 à 31, qui comprend l'étape consistant à détecter une pulsation à l'intérieur dudit doigt (7) avec ledit microprocesseur (16), contrôler le microprocesseur (16) pour qu'il accepte uniquement les mesures qui ont été prises au même moment par rapport à ladite pulsation.

40. Procédé selon l'une quelconque des revendications 29 à 31, comprenant les étapes consistant à contrôler le microprocesseur (16) pour balayer le spectre d'intérêt en entier de nombreuses fois par seconde à plusieurs reprises comprises dans la plage allant d'environ 8 à environ 64 reprises et à faire une moyenne des résultats avec ledit microprocesseur.

41. Procédé selon l'une quelconque des revendications 29 à 31, comprenant l'étape consistant à mesurer le niveau de concentration des constituants qui sont sélectionnés, sans s'y limiter, dans le groupe constitué par : un acide aminé, l'azote, l'oxygénation sanguine, le dioxyde de carbone, le cortisol, la créatine, la créatinine, le glucose, les corps cétoniques, les lipides, les graisses, l'urée, les acides aminés, les acides gras, l'hémoglobine glycosée, le cholestérol, l'alcool, le lactate, $Ca^{++}$, $K^+$, $Cl^-$, $HCO_3^-$, et $HPO_4^-$.

FIGURE 1

EP 0 630 203 B1

FIGURE 2

7 FINGER

THERMOCOUPLE—26

FINGER RECEPTOR—6

OSRAM LAMP—1

LENS-3

FILTER—2

LENS 4

30 MIRROR

25 FILTER SHUTTER

30 MIRROR

31 LENS

DUAL SHUTTER 32

30 MIRROR

SCATTERED LIGHT

SLIT APERTURE 10

30 — MIRROR

9 LENS

SHUTTER 24

GRATING 12

11 LENS

50mm LENS 13

12mm LENS 14

15 SENSOR

30 MIRROR

A/D CONVERTER 17

DISPLAY 18

PRINTER 19

MICROPROCESSOR 16

KEYBOARD 20